Europäisches Patentamt

European Patent Office

Office européen des brevets

(11) Publication number: **0 042 939**
**B1**

# (12) EUROPEAN PATENT SPECIFICATION

(45) Date of publication of patent specification: **16.04.86**

(51) Int. Cl.⁴: **A 61 M 1/34**

(21) Application number: **81103504.7**

(22) Date of filing: **08.05.81**

(54) Hemofiltration system.

(30) Priority: **27.06.80 SE 8004795**

(43) Date of publication of application:
**06.01.82 Bulletin 82/01**

(45) Publication of the grant of the patent:
**16.04.86 Bulletin 86/16**

(84) Designated Contracting States:
**AT BE CH DE FR GB IT LI LU NL SE**

(56) References cited:
**FR-A-2 190 474**
**FR-A-2 276 835**
**FR-A-2 288 529**
**FR-A-2 397 197**
**GB-A-2 058 604**
**US-A-3 579 441**
**US-A-4 081 372**
**US-A-4 134 834**
**US-A-4 191 182**

(73) Proprietor: **Gambro Lundia AB**
**Box 10101**
**S-220 10 Lund (SE)**

(72) Inventor: **Shaldon, Stanley**
**Villa 86 Rue de Grézac**
**F-34100 Montpellier (FR)**
Inventor: **Gullberg, Claes-Ake**
**380 E. Dexter**
**Covina, CA 91723 (US)**
Inventor: **Larsson, Lars-Ake Lennart**
**Odlarevägen 66**
**S-241 00 Löddeköpinge (SE)**

(74) Representative: **Boberg, Nils Gunnar Erik**
**Gambro AB Patent Department Box 10101**
**S-220 10 Lund (SE)**

EP 0 042 939 B1

Courier Press, Leamington Spa, England.

## Description

### Technical Field

The present invention relates to a hemofiltration system comprising a hemofilter for withdrawal of plasma from a patient and replacement liquid preparing means including connection means for connection to a normal water conduit, purifying means for purifying supplied water, and mixing means for mixing said water with a concentrate for preparing the replacement liquid intended to replace the withdrawn plasma.

### The State of the Art

In hemofiltration, blood from a patient is passed through a hemofilter on one side of a semipermeable membrane. Through this membrane, plasma water from the blood is ultrafiltered. At the same time toxics for example urea included in the blood are removed. The amount of ultrafiltrate is, for a normal patient, usually of the order of magnitude of 20 litres and most of it must, therefore, be replaced by a replacement or infusate solution which consists of water with an addition of suitable substances for realising an isotonic solution.

### Description of the Present Invention

The object of the present invention is instead to realise a hemofiltration system which in itself includes means for preparing the replacement liquid. The system is here characterized by a filter through which the prepared liquid is supplied to the blood before said blood is returned to the patient under guaranteed sterile conditions.

Preferably, this means for purifying the supplied water consists of a module for reverse osmosis. Such modules are per se known to the person skilled in this art and need not, therefore, be described in any detail. However, alternative methods of purification may also be used.

The function of the above-mentioned module for reverse osmosis may be improved if a preheater is coupled-in ahead of the module for heating the water emanating from the water supply conduit.

This means for purifying the supplied water should suitably further contain a cartridge with activated charcoal for adsorption of chlorine and pyrogenes.

Suitably, use is made, for the above-mentioned filter, of a type which is substantially the same as that used for the hemofiltration proper, that is a hemofilter including a semipermeable membrane. As a result, it will be assured that the filter allows the passage of desired substances, that is replacement substances for those substances which erroneously were lost in the hemofiltration.

Preferably, the system according to the invention includes means for measuring both the ultrafiltrate and the replacement liquid, as well as means for comparing the measured values for the purposes of calculating suitable weight-reduction for the patient.

The output sides of both the above-mentioned filter and the hemofiltration filter proper may suitably be connected to a common drip chamber whence a conduit leads the mixture of filtrate to the infusion site on the patient. Hereby, the infusion will be facilitated, at the same time as a simple conduit set is made possible which is suitable for once-over use.

A particularly simple system will be obtained if use is made, for the above-mentioned filter, of the hemofiltration filter itself which to this end is operative to be driven intermittently for alternating ultrafiltration of the plasma from the blood and filtration of the replacement liquid into the blood. The means for preparing the replacement may here be connected to a connection socket on the hemofilter which is disposed also to serve as an outlet or drain for the ultrafiltrate or plasma from the blood. Valves are here disposed alternatingly to connect the socket to the above-mentioned means for preparing the replacement liquid and to an outlet or receptacle point for ultrafiltrate from the blood.

If batch preparation of replacement liquid is desired, the system may be provided with means for shutting-down the preparation when the desired amount of replacement liquid is available. Alternatively, the system for preparation of a certain amount of replacement liquid may be disposed in a first container and the system may be provided with means for switching the supply of replacement liquid from this first container to a second container and vice versa, when the desired amount of liquid is to be found in each respective container.

Since the system according to the present invention is intended directly to be connected to a patient, it should suitably be coordinated with a safety system for control of the above-mentioned filter. This safety system is described and claimed in our divisional application EP—A—0087171.

### Brief Description of the Accompanying Drawings

The present invention will be described in greater detail below with reference to the accompanying drawings which illustrate four different hemofiltration systems according to the invention.

Fig. 1 shows a first hemofiltration system with continuous preparation of replacement liquid.

Fig. 2 shows a second hemofiltration system with intermittent preparation of replacement liquid and with intermittent supply thereof to the patient.

Fig. 3 shows a third hemofiltration system according to the invention with batchwise preparation of the replacement liquid. When a portion has been prepared, this is continuously supplied to the patient.

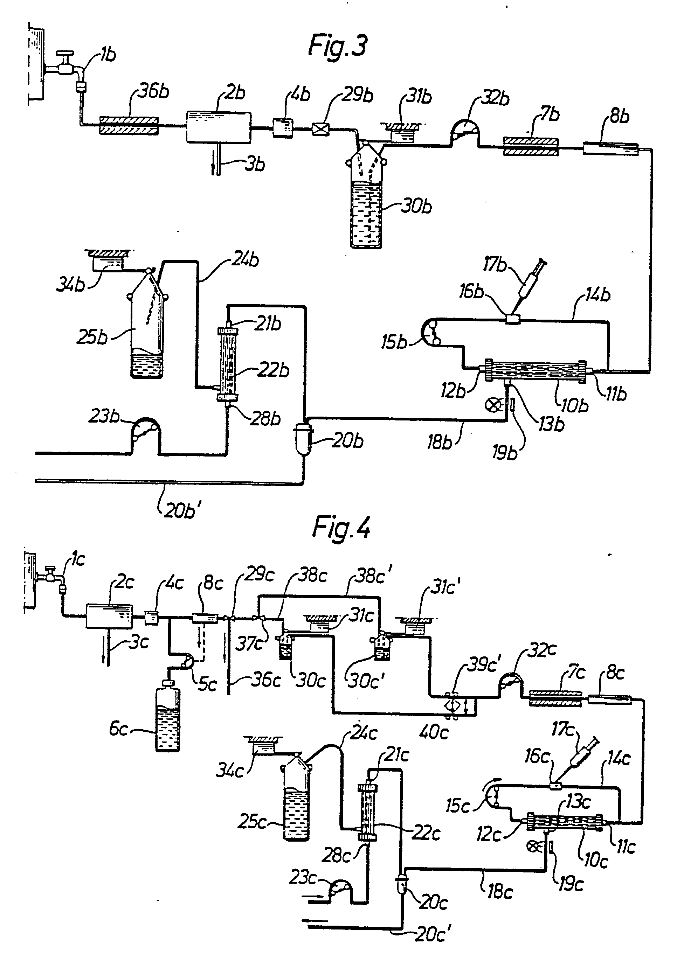

Finally, Fig. 4 shows a fourth hemofiltration system with continuous preparation of replacement liquid which may be divided into portions which are then continuously supplied to the patient.

Hemofiltration with Continuous Preparation of Replacement Liquid

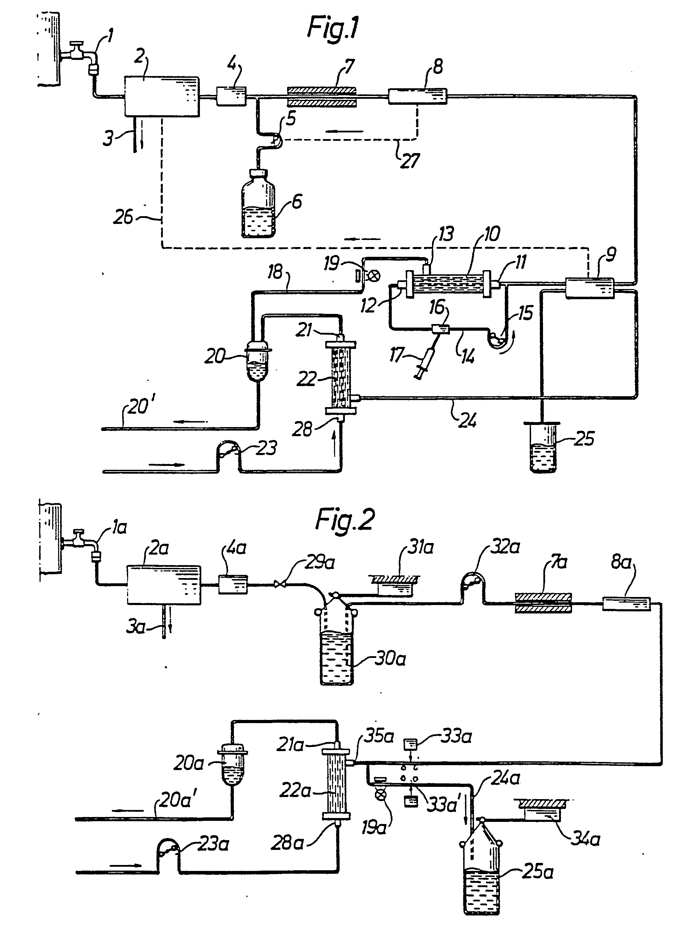

In Fig. 1, a water conduit connection is designated 1, for example a normal water faucet. The water is led thence into a module 2 for reverse osmosis, whence waste water is drained off via a conduit 3. The purified water is instead passed through a container 4 which is filled with activated charcoal and is intended for adsorption of chlorine and pyrogenes. Concentrate for the replacement liquid is dosed by means of a pump 5 from a container 6 in order to realise an isotonic solution. This solution is led through a heating device 7 and a conductivity metre 8 which in its turn controls the pump 5.

The prepared solution is then led through a flowmetre 9 to a filter 10 with an inlet 11 and an outlet 12 for the liquid which is to be filtered, as well as an outlet 13 for the filtrate. The filter 10 may, for example, consist of a normal hemofilter containing fibres of a semipermeable material.

A recirculation circuit 14 is connected to the in and outlets 11 and 12 and has a pump 15 and an injection site 16. At the injection site 16, an indicator, for example blue dextrane may be injected by means of a normal injection syringe 17. The outlet 13 is connected to a conduit 18 which includes a photocell sensing device 19. Should a leak occur in the filter 10, this would, thus, be discovered directly such that measures may be taken.

The conduit 18 discharges into a drip chamber 20 to which the outlet side 21 of a hemofilter 22 is also connected. From the drip chamber 20, the mixture of replacement liquid and blood is led via the conduit 20' to the patient.

The hemofilter 22 is fed by means of a pump 23 with blood from the patient (not shown). Plasma or ultrafiltrate is removed in a conventional manner through a conduit 24 by the intermediary of the flowmetre 9 to a receptacle site 25. The broken line 26 intimates how the flowmetre 9 may be disposed to control the module 2 for reverse osmosis. In the same manner, the line 27 intimates that the conductivity metre 8 may be disposed to control the pump 5 for pumping concentrate from the container 6 to the main conduit. Finally, the blood inlet for the hemofilter 22 is designated 28.

Hemofiltration with Intermittent Supply of Replacement Liquid and Batchwise Preparation Thereof

The system according to Fig. 2 corresponds in essential details to that according to Fig. 1. Corresponding details have, therefore, been given the same reference numerals, but with the addition of an *a* in Fig. 2. Thus, the following details recur:

1a — connection point for water conduit water, for example a normal water faucet
2a — module for reverse osmosis
3a — outlet for impure water
4a — cartridge with activated charcoal
29a — shut-off valve
30a — container for replacement liquid

31a — scales for replacement liquid
32a — pump for replacement liquid
7a — heating device
8a — conductivity metre
33a — valve
33a' — valve
24a — conduit for hemofiltrate
19a — photocell sensing device
25a — receptacle container for hemofiltrate
34a — scales for hemofiltrate
22a — hemofilter
28a — blood inlet for hemofilter
21a — blood outlet for hemofilter
35a — inlet for replacement liquid and outlet for hemofiltrate
20a — drip chamber
20a' — return conduit to patient
23a — blood pump

The system according to Fig. 2 differs from that in Fig. 1 in two essential respects. First, the replacement liquid is prepared batchwise in the container 30a. Here, the concentrate may either be supplied to the container initially or according as the process progresses. If necessary, the container may be supplemented with suitable agitator devices. When there is a suitable amount in the container 30a, the valve 29a is shut-off. The hemofiltration proper may then be commenced. Here, the hemofilter 22a operates intermittently in such a manner that, for a certain period of time, for example, one minute, it works as a pure hemofilter with the valve 33a closed and the valve 33a' open. Then, these valves are switched and replacement liquid is supplied through the filter 22a proper by being introduced under pressure through the inlet 35a. Such alternate supply of replacement liquid and ultrafiltration of the blood continues then until such time as the desired amount has been removed from the container 30a and a corresponding amount, or preferably a slightly greater amount, has been supplied to the container 25a. In the latter case, the patient will undergo a desired reduction in weight.

Hemofiltration with Batchwise Preparation of Replacement Liquid

The system according to Fig. 3 corresponds also in essential parts to the systems according to Figs. 1 and 2. Hence the same reference numerals have also been used here, but with the addition of a *b*. Thus, the following details are to be found in the system:

1b — connection point for water conduit water, for example a normal water faucet
36b — preheater
2b — module for reverse osmosis
3b — outlet for impure water
4b — cartridge with activated charcoal
29b — shut-off valve
30b — container for replacement liquid
31b — scales for replacement liquid
32b — pump for replacement liquid
7b — heating device
8b — conductivity metre
10b — filter

11b — inlet for filter
12b — outlet for filter
13b — outlet for filtrate
14b — recirculation circuit
15b — recirculation pump
16b — injection site for indicator
17b — injection syringe for indicator
18b — conduit
19b — photocell sensing device
20b — drip chamber
20b' — return conduit to patient
21b — blood outlet for hemofilter
22b — hemofilter
28b — blood inlet for hemofilter
23b — pump for blood
24b — outlet conduit for the hemofiltrate
25b — receptacle container for the hemofiltrate
33b — scales for the hemofiltrate

The preparation of the replacement liquid according to Fig. 3 takes place in the same manner as that in Fig. 2, that is batchwise. The supply to the patient takes place, on the other hand, substantially in the same manner as in the system according to Fig. 1, that is through a filter 10b with a specially built-in safety system and then a drip chamber 20b. The ultrafiltrate is then measured in the same manner as in the system according to Fig. 2.

## Hemofiltration with Continuous Batchwise Preparation of Replacement Liquid and Continuous Supply to the Patient

The system according to Fig. 4 also corresponds in essential details to the systems according to Figs. 1 to 3. The same reference numerals have, therefore, also been used here, but with the addition of the letter c. Thus, the following details are present in this system:

1c — connection point for water conduit water, for example a normal water faucet
  2c — module for reverse osmosis
  3c — outlet for impure water
  4c — cartridge with activated charcoal
  5c — pump
  6c — container for concentrate
  8c — conductivity metre
  29c — shut-off valve
  36c — discharge conduit for liquid of incorrect composition
  37c — valve
  38c — conduit to a first dosage container
  30c — first dosage container
  38c' — conduit to second dosage container
  30c' — second dosage container
  31c — scales for first dosage container
  31c' — scales for second dosage container
  39c — valve
  40c — valve
  32c — pump for replacement liquid
  7c — heating device
  8c — conductivity metre
  10c — filter
  11c — inlet
  12c — outlet
  13c — outlet for the filtrate

14c — recirculation circuit
15c — recirculation pump
16c — injection site for indicator
17c — injection syringe for indicator
18c — conduit
19c — photocell sensing device
20c — drip chamber
20c' — return conduit to the patient
21c — blood outlet for hemofilter
22c — hemofilter
28c — blood inlet for hemofilter
23c — blood pump
24c — outlet conduit for the hemofiltrate
25c — receptacle container for the hemofiltrate
34c — scales for the hemofiltrate

The system according to Fig. 4 may be described as a combination of earlier described systems. Replacement liquid is here continuously prepared by means of details 1c, 2c, 3c, 4c, 5c, 6c and 8c. Should the conductivity be incorrect, the liquid is removed via the conduit 36c, thanks to the switch valve 29c. On the other hand, if the conductivity metre 8c shows that the right composition has been obtained, the liquid is passed via the valve 37c and the conduit 38c to a first dosage container 30c which is weighed by means of scales 31c. When a suitable amount of liquid has arrived at this container, it is fed further, at the same time as newly-prepared liquid instead is passed via the valve 37c and the conduit 38c' to a second dosage container 30c' which is weighed by means of scales 31c'. Here, the valve 39c is closed, whereas the previously prepared is passed via the valve 40c and the pump 32c through the heating device 7c and the conductivity metre 8c, via the filter 10c and the drip chamber 20c to the patient. The filter 10c is, here, provided with a safety system of the above-described type. When the container 30c has been emptied, new liquid has been prepared in the container 30c'. The valves 37c, 39c and 40c may here be switched for discharge of the newly-prepared liquid. During the entire time, blood is continuously pumped by means of the pump 23c through the hemofilter 22c and via the drip chamber 20c back to the patient. At the same time, hemofiltrate is removed via the conduit 24c and is collected in the container 25c which is weighed by means of scales 34c.

Naturally, the present invention is not restricted merely to the above-described Examples, but may be varied within the scope of the appended claims. For example, the different details included in the systems may be varied and combined in a number of ways different from those described above. The charcoal containers 4, 4a, 4b and 4c may for instance be placed before the reverse osmosis modules 2, 2a, 2b and 2c, respectively. Before those reverse osmosis modules it is also possible to place softening devices, if the water from the tap is too hard.

## Claims

1. Hemofiltration system comprising a hemofilter (22) for withdrawal of plasma from a patient and replacement liquid preparing means (1—8) including connection means (1) for connection to a normal water conduit, purifying means (2—4) for purifying supplied water, and mixing means (5, 6, 8) for mixing said water with a concentrate for preparing the replacement liquid intended to replace the withdrawn plasma, characterized by a filter (10, 22a, respectively) through which the prepared liquid is filtered and supplied to the blood before said blood is returned to the patient.

2. System according to claim 1, characterized in that said purifying means (2—4) for purifying the supplied water include a module (2) for reverse osmosis.

3. System according to claim 2, characterized by a preheater (36b) coupled in for said module (2b) for reverse osmosis.

4. System according to claim 2, characterized in that said purifying means (2—4) for purifying the supplied water further comprises a cartridge (4) containing activated charcoal.

5. System according to any one of claims 1 to 3, characterized in that, as said filter (10, 22, respectively) use is made of a filter of substantially the same type as that (22) which is used for the hemofiltration proper, that is a hemofilter including a semipermeable membrane.

6. System according to any one of the preceding claims, characterized by measuring means (9; 31a; 34a; 31b; 34b; 31c; 31c', 34c) for measuring both ultrafiltrate and replacement liquid.

7. System according to any one of the preceding claims, characterized in that the output sides (13, 21) of both said filter (10) and the hemofiltration filter (22) are connected to a common drip chamber (20), whence a conduit (20') passes for the mixture of filtrate to the site of infusion to the patient.

8. System according to any one of the preceding claims, characterized in that, as said filter, use is made of the hemofiltration filter (22a) proper, which is disposed to be driven intermittently for alternating ultrafiltration of plasma from the blood and filtration of replacement liquid.

9. System according to claim 8, characterized in that said preparing means for preparing replacement liquid are connected to a connection socket (35a) of the hemofilter (22a) which is also disposed to serve as an outlet for ultrafiltrate or plasma from the blood, valves (33a, 33a') being disposed alternatingly to connect the socket (35) to said means (1a—8a) for preparing replacement liquid and to an outlet (24a) or receptacle site (25a) for ultrafiltrate from the blood.

10. System according to any one of claims 1 to 7, characterized by preparing means (1a, 2a, 3a, 4a, 29a, 30a, 31a) for preparing a certain amount of replacement liquid, and means (29a) for shutting down the preparation when a desired amount of replacement liquid is available.

11. System according to any one of claims 1 to 7, characterized by preparing means (1c, 2c, 3c, 4c, 5c, 6c, 8c, 29c, 36c, 37c, 38c) for preparing a certain amount of replacement liquid in first container (30c), and means (37c) for switching the supply of replacement liquid from this first container (30c) to a second container (30c') and vice versa, when the desired amount of liquid is in each respective container.

12. System according to any one of the preceding claims, characterized by a safety system coordinated with said filter (10) for detecting possible leaks in the filter (10, 22a, respectively) through which the prepared liquid is supplied to the blood.

**Patentansprüche**

1. Hämofiltrationssystem mit einem Hämofilter (22) für die Entnahme von Plasma von einem Patienten und mit Einrichtungen (1—8) zur Herstellung von Austauschflüssigkeit einschließlich Verbindungseinrichtungen (1) für die Verbindung mit einer Normalwasserleitung, Reinigungseinrichtungen (2—4) zur Reinigung von zugeführtem Wasser und Mischeinrichtungen (5, 6, 8) zum Mischen des Wassers mit einem Konzentrat zur Herstellung der Austauschflüssigkeit, die für den Ersatz des entnommenen Plasmas bestimmt ist, gekennzeichnet durch ein Filter (10 bzw. 22a), durch welches die hergestellte Flüssigkeit filtriert und dem Blut zugeführt wird, bevor dieses Blut zu dem Patienten zurückgefuhrt wird.

2. System nach Anspruch 1, dadurch gekennzeichnet, daß die Reinigungseinrichtungen (2—4) für das Reinigen des zugeführten Wassers einen Modul (2) für umgekehrte Osmose enthalten.

3. System nach Anspruch 2, gekennzeichnet durch einen eingeschalteten Vorerhitzer (36b) für diesen Modul (2b) für umgekehrte Osmose.

4. System nach Anspruch 2, dadurch gekennzeichnet, daß die Reinigungseinrichtungen (2—4) zum Reinigen des zugeführten Wassers zusätzlich eine Aktivkohle enthaltende Patrone (4) aufweisen.

5. System nach einem der Ansprüche 1 bis 3, dadurch gekennzeichnet, daß als das Filter (10 bzw. 22) ein Filter von im wesentlichen der gleichen Art wie jenes (22) verwendet wird, das für die eigentliche Hämofiltration verwendet wird, d.h. ein Hämofilter mit einer semipermeablen Membran.

6. System nach einem der vorausgehenden Ansprüche, gekennzeichnet durch Meßeinrichtungen (9; 31a; 34a; 31b; 34b; 31c; 31c', 34c) zum Messen von Ultrafiltrat und Austauschflüssigkeit.

7. System nach einem der vorausgehenden Ansprüche, dadurch gekennzeichnet, daß die Ausgangsstellen (13, 21) sowohl des Filters (10) als auch des Hämofiltrationsfilters (22) mit einer gemeinsamen Tropfkammer (20) verbunden sind, von wo eine Leitung (20') für das Filtratgemisch zu der Infusionsstelle zu dem Patienten geht.

8. System nach einem der vorausgehenden Ansprüche, dadurch gekennzeichnet, daß als das Filter das eigentliche Hämofiltrationsfilter (22a) verwendet wird, das so ausgebildet ist, daß es für abwechselnde Ultrafiltration von Plasma aus dem Blut und Filtration von Austauschflüssigkeit betrieben wird.

9. System nach Anspruch 8, dadurch gekennzeichnet, daß die Herstellungseinrichtungen zur Herstellung von Austauschflüssigkeit mit einer Verbindungsbuchse (35a) des Hämofilters (22a) verbunden sind, die auch so ausgebildet ist, daß sie als ein Auslaß für Ultrafiltrat oder Plasma aus dem Blut dient, wobei Ventile (33a, 33a') alternativ zur Verbindung der Buchse (35) mit den Einrichtungen (1a bis 8a) zur Herstellung von Austauschflüssigkeit und mit einem Auslaß (24a) oder einer Aufnahmestelle (25a) für Ultrafiltrat aus dem Blut ausgebildet sind.

10. System nach einem der Ansprüche 1 bis 7, gekennzeichnet durch Herstellungseinrichtungen (1a, 2a, 3a, 4a, 29a, 30a, 31a) zur Herstellung einer bestimmten Menge von Austauschflüssigkeit und Einrichtungen (29a) zum Abschalten der Herstellung, wenn eine erwünschte Menge an Austauschflüssigkeit verfügbar ist.

11. System nach einem der Ansprüche 1 bis 7, gekennzeichnet durch Herstellungseinrichtungen (1c, 2c, 3c, 4c, 5c, 6c, 8c, 29c, 36c, 37c, 38c) zur Herstellung einer bestimmten Menge von Austauschflüssigkeit in einem ersten Behälter (30c) und Einrichtungen (37c) zum Umschalten der Zufuhr von Austauschflüssigkeit von diesem ersten Behälter (30c) zu einem zweiten Behälter (30c') und umgekehrt, wenn die erwünschte Flüssigkeitsmenge sich in dem jeweiligen Behälter befindet.

12. System nach einem der vorausgehenden Ansprüche, gekennzeichnet durch ein mit diesem Filter (10) koordiniertes Sicherheitssystem zur Ermittlung möglicher Leckagen in dem Filter (10 bzw. 22a), durch welches die hergestellte Flüssigkeit dem Blut zugeführt wird.

**Revendications**

1. Système d'hémofiltration comprenant un hémofiltre (22) pour l'extraction du plasma d'un patient et des moyens de préparation (1—8) d'un liquide de compensation comprenant des moyens de raccordement (1) pour le raccordement à une prise d'eau normale, des moyens de purification (2—4) pour purifier l'eau fournie, et des moyens mélangeurs (5, 6, 8) pour mélanger ladite eau avec un concentré pour préparer le liquide de compensation destiné à remplacer le plasma extrait, caractérisé en ce qu'il comprend un filtre (10, 22a, respectivement) dans lequel le liquide préparé est filtré et ajouté au sang avant que celui-ci ne soit restitué au patient.

2. Système suivant la revendication 1, caractérisé en ce que les moyens de purification (2—4) pour purifier l'eau fournie comprennent un module (2) d'osmose inverse.

3. Système suivant la revendication 2, caractérisé en ce qu'un appareil de préchauffage (36b) est adjoint au module (2b) d'osmose inverse.

4. Système suivant la revendication 2, caractérisé en ce que les moyens (2—4) de purification de l'eau fournie comprennent en outre une cartouche (4) contenant du charbon activé.

5. Système suivant l'une quelconque des revendications 1 à 3, caractérisé en ce que, pour les filtres (10, 22, respectivement) il est fait usage d'un filtre sensiblement du même type que le filtre (22) utilisé pour l'hémofiltration proprement dite, c'est-à-dire un hémofiltre à membrane semi-perméable.

6. Système suivant l'une quelconque des revendications précédentes, caractérisé en ce qu'il comprend des moyens de mesure (9; 31a; 34a; 31b; 34b; 31c; 31c'; 34c) pour mesurer à la fois l'ultrafiltrat et le liquide de compensation.

7. Système suivant l'une quelconque des revendications précédentes, caractérisé en ce que les orifices de sortie (13, 21) du filtre (10) et du filtre d'hémofiltration (22) sont reliées à une chambre d'écoulement commune (20), d'où part une tubulure (20') pour la restitution du mélange de filtrat au point d'injection au patient.

8. Système suivant l'une quelconque des revendications précédentes, caractérisé en ce que, pour le filtre, il est fait usage d'un filtre d'hémofiltration (22a) proprement dit, qui est disposé de façon à fonctionner par intermittence pour faire alterner l'ultrafiltration du plasma sanguin et la filtration du liquide de compensation.

9. Système suivant la revendication 8, caractérisé en ce que les moyens pour la préparation du liquide de compensation sont reliés à une prise (35a) de l'hémofiltre (22a) qui peut également servir d'orifice de sortie pour l'ultrafiltrat du plasma sanguin, des valves (33a, 33a') étant agencées de manière à relier alternativement la prise (35) auxdits moyens (1a—8a) de préparation du liquide de compensation et à un orifice de sortie (24a) ou à un site de collecte (25a) pour l'ultrafiltrat du sang.

10. Système suivant l'une quelconque des revendications 1 à 7, caractérisé en ce qu'il comprend des moyens de préparation (1a, 2a, 3a, 4a, 29a, 30a, 31a) pour la préparation d'une certaine quantité de liquide de compensation, et des moyens (29a) pour interrompre la préparation lorsque la quantité voulue de liquide de compensation est atteinte.

11. Système suivant l'une quelconque des revendications 1 à 7, caractérisé en ce qu'il comprend des moyens de préparation (1c, 2c, 3c, 4c, 5c, 6c, 8c, 29c, 36c, 37c, 38c) pour la préparation d'une certaine quantité de liquide dans un premier récipient (30c), et des moyens (37c) pour commuter l'alimentation en liquide de compensation de ce premier récipient (30c) à un deuxième récipient (30c') et vice-versa, lorsque la quantité voulue de liquide se trouve dans chaque récipient respectif.

12. Système suivant l'une quelconque des revendications précédentes, caractérisé en ce qu'un système de sécurité est associé au filtre (10) pour détecter les fuites éventuelles dans le filtre (10, 22a respectivement) à travers lequel le liquide préparé est ajouté au sang.

Fig.1

Fig.2

**Fig.3**

**Fig.4**